(19) Europäisches Patentamt
European Patent Office
Office européen des brevets

(11) Veröffentlichungsnummer : **0 054 174**
**B1**

(12) # EUROPÄISCHE PATENTSCHRIFT

(45) Veröffentlichungstag der Patentschrift : **20.08.86**

(51) Int. Cl.[4] : **A 61 K 7/06**, A 61 K 7/48

(21) Anmeldenummer : **81109351.7**

(22) Anmeldetag : **30.10.81**

(54) **Topische kosmetische Zubereitungen zur Behandlung von stark fettendem Haar und seborrhoeischer Haut.**

(30) Priorität : **13.12.80 DE 3047106**

(43) Veröffentlichungstag der Anmeldung : **23.06.82 Patentblatt 82/25**

(45) Bekanntmachung des Hinweises auf die Patenterteilung : **20.08.86 Patentblatt 86/34**

(84) Benannte Vertragsstaaten : **AT BE CH DE FR GB IT LI LU NL SE**

(56) Entgegenhaltungen :
**DE-A- 1 617 477**
**DE-A- 2 721 394**
**FR-A- 1 547 573**
**FR-A- 2 383 662**
**FR-A- 2 480 600**
**US-A- 4 201 235**
**CHEMICAL ABSTRACTS, vol. 92, no. 12, 24. Mai 1980, Seite 336, Nr. 99449n, Columbus, Ohio, USA**
**JOURNAL OF THE CHEMICAL SOCIETY, April 1959, Seiten 2157-2168, London, G.B. M. BARASH et al.: "Chemical constitution and amoebicidal action. Part III. Synthesis of an analogue of emetine and two stereoisomers of De-ethylemetine"**
**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Möller, Hinrich, Dr.**
**Schumannstrasse 11**
**D-4019 Monheim (DE)**
Erfinder : **Wallat, Siegfried, Dr.**
**Hasenstrasse 7**
**D-4019 Monheim (DE)**
Erfinder : **Bartnik, Friedheim, Dr.**
**Melanchthonstrasse 11**
**D-4000 Düsseldorf (DE)**
Erfinder : **Pittermann, Wolfgang, Dr.**
**Schwarzbachstrasse 33**
**D-4000 Düsseldorf 12 (DE)**

**EP 0 054 174 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind topische kosmetische Zubereitungen,die der Bekämpfung des fettigen und unästhetischen Aussehens der Haare und der Haut dienen.

Die übermäßig starke Absonderung der Talgdrüsen der Kopfhaut verleiht dem Haar ein fettiges Aussehen, das im allgemeinen als wenig ästhetisch angesehen wird. Es hat daher nicht an Bestrebungen gefehlt, durch geeignete Mittel die Sekretion der Talgdrüsen zu normalisieren, um dem Haar wieder sein gesundes Aussehen zu geben. Zur Behebung dieser seborrhoeischen Erscheinungen ist bereits eine große Zahl synthetischer Produkte, insbesondere schwefelhaltige Verbindungen eingesetzt worden, ohne daß wirklich befriedigende Ergebnisse im Hinblick auf Wirksamkeit oder anwendungstechnische Eigenschaften erzielt werden konnten.

Aus FR 23 83 662 sind Kompositionen für den kosmetischen Gebrauch bekannt, die Oleylester der Formel

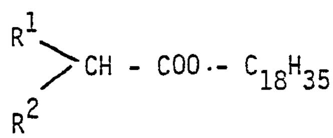

enthalten, wobei $R^1$ u. a. Phenyl, Benzyl oder Phenethyl sein kann. Diesen Verbindungen wird eine Wirksamkeit gegen Corynebacterium Acne bzw. gegen die bei Akne auftretenden Entzündungen, Pusteln und dergleichen zugeschrieben.

Es wurde nun gefunden, daß topische kosmetische Zubereitungen mit einem Gehalt an Verbindungen der allgemeinen Formel

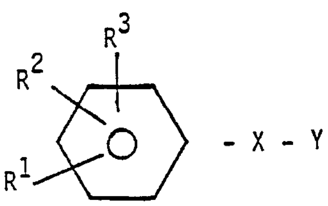

In der $R^1$, $R^2$ und $R^3$ unabhängig voneinander für ein Wasserstoffatom, eine Hydroxylgruppe eine $OR^4$-Gruppe, wobei $R^4$ einen Alkylrest mit 1-6 Kohlenstoffatomen bedeutet, oder zwei der Reste $R^1$, $R^2$ und $R^3$ für eine Methylendioxygruppe stehen, wobei die Reste $R^1$, $R^2$ und $R^3$ nicht gleichzeitig Wasserstoff bedeuten, X für eine Alkylengruppe mit 1-3 Kohlenstoffatomen in der Kette und Y für eine Gruppe $CONR^5R^6$ oder $COOR^7$ stehen, worin $R^5$ und $R^6$ ein Wasserstoffatom, einen Alkylrest mit 1-6 Kohlenstoffatomen, einen Benzyl- oder 3,4-Dimethoxybenzylrest und $R^7$ einen Alkylrest mit 1-6 Kohlenstoffatomen darstellen, oder an
2-(3,4-Dimethoxy-benzyl)-acetessigsäure-methylester
4-(3,4-Methylendioxy-benzyloxy)-phenylessigsäureethylester
N-[β-(3,4-Dimethoxy-phenyl)-ethyl]-3,4-dimethoxy-phenyl-acetamid oder
N-[β-(3,4-Dimethoxy-phenyl)-ethyl]-N-methyl-3,4-dimethoxy-phenylacetamid eine besonders bemerkenswerte Wirksamkeit bei der Behandlung von seborrhoeischer Haut und stark fettendem Haar aufweisen.

Die in den erfindungsgemäßen Zubereitungen einzusetzenden Verbindungen sind selbst oder in Form der Vorprodukte literaturbekannt und zum Teil Käuflich zu erwerben. Soweit nur die den Verbindungen zugrunde liegenden Arylalkansäuren käuflich sind, lassen sich die erfindungsgemäß einzusetzenden Verbindungen auf einfache Weise nach allgemein bekannten Verfahren der organischen Chemie herstellen. Aus den Alkoxyarylalkansäuren sind die entsprechenden Hydroxyverbindungen oft auch durch partielle oder vollständige Demethylierung mit Jodwasserstoff erhältlich. Dieses Herstellungsverfahren ist auch zur Gewinnung der Hydroxyarylalkansäureamide anwendbar. Zu Benzyloxysubstituierten Arylalkansäurederivaten gelangt man durch Reaktion der Hydroxy-substituierten Ester mit gegebenenfalls substituierten Benzylhalogeniden. Danach läßt sich die Carbonsäurefunktion in allgemein bekannter Weise variieren. Alkoxybenzylacetessigsäureester lassen sich durch Alkylierung der entsprechenden unsubstituierten Ester mit Alkoxybenzylhalogeniden herstellen. Die entsprechenden Ester oder Amide sind nach den üblichen Methoden der organischen Chemie aus den Carbonsäuren herstellbar.

Als den in den erfindungsgemäßen Zubereitungen einzusetzenden Verbindungen zugrunde liegende Arylalkansäuren sind zum Beispiel 2-Hydroxy-, 3-Hydroxy-, 4-Hydroxy-, 2-Methoxy-, 3-Methoxy-, 4-Methoxy-, 4-Ethoxy-, 4-Isopropyloxy-, 4-Hexyloxy-, 4-Benzyloxy-, 4-(3,4-Methylendioxy-benzyloxy)-phenylessigsäure, 2-(p-Hydroxy-phenyl)-, 3-(p-Hydroxy-phenyl)-, 4-(p-Hydroxy-phenyl)-buttersäure, 4-Hydroxy-3-methoxy-, 2,4-Dihydroxy-, 2,5-Dihydroxy-, 3,4-Dihydroxy-, 3,5-Dihydroxy-, 2,4-Dimethoxy-, 3,4-Dimethoxy-, 3,4-Methylendioxy-, 3-Methoxy-4-ethoxy-, 3-Methoxy-4-benzyloxy-, 3,4,5-Trihydroxy-, 3,4,5-Trimethoxy-phenylessigsäure, 2-(3,4-Dimethoxy-benzyl)-, 2-(3,4-Methylendioxybenzyl)-acetessigsäure, 2-(p-Methoxy-phenyl)-2-methyl-, 3-(3,4-Dimethoxy-phenyl)-propionsäure zu nennen.

Als Alkoholkomponente für die Herstellung der erfindungsgemäß einzusetzenden Arylalkansäureester kommen zum Beispiel Methanol, Ethanol, Propanol, 2-Propanol, Butanol, Isobutanol, tert. Butanol, Pentanol, Hexanol, Benzylalkohol, 3-4-Dimethoxybenzylalkohol in Frage.

Als Aminkomponente für die Herstellung der erfindungsgemäß einzusetzenden Hydroxy- bzw. Alkoxyarylalkansäureamide sind zum Beispiel Ammoniak, Methyl-, Ethyl-, Propyl-, Butyl-, Hexyl-, Dimethyl-, Diethyl-, Dipropyl-, Dibutyl-, Benzyl-, 3,4-Dimethoxybenzyl-amin zu nennen.

Die erfindungsgemäßen kosmetischen Zubereitungen stellen Lösungen der einzusetzenden wirksamen Verbindungen vorgenannter allgemeiner Formel in Wasser, in Alkoholen, in wäßrig-alkoholischen Mischungen, in Ölen, Suspensionen in Gelen, Emulsionen, Salben, Pasten, Aerosolen dar. Grundsätzlich eignen sich fast alle kosmetischen Mittel zur Behandlung von Haut und Haaren zur Einarbeitung der Verbindungen vorgenannter allgemeiner Formel, um diesen auf diesem Wege antiseborrhoeische Eigenschaften zu verleihen. So können die erfindungsgemäßen Kosmetischen Zubereitungen in Form von Haarlacken, Well-Lotionen und Haarspülungen vorliegen. Derartige Präparate enthalten neben den in Lösung oder Suspension vorliegenden Verbindungen vorgenannter allgemeiner Formel gegebenenfalls oberflächenaktive Mittel, Cellulosederivate, kationische Verbindungen, Vitamine, Harze, Farbstoffe und Parfumöle. Die alkoholischen oder wässrigalkoholischen Lösungenkönnen dabei im Gemisch mit geeigneten Treibgasen auch in Form von Aerosolen zum Einsatz gelangen. Eine weitere Anwendungsform der erfindungsgemäßen Zubereitungen stellen solche Produkte dar, die auf die Haut aufgetragen werden und im allgemeinen in Form von Cremes, Milchpräparaten, Gelen, Pasten vorliegen. Neben den Verbindungen vorgenannter allgemeiner Formel enthalten diese Zubereitungen als übliche Bestandteile Wasser, Lösungsmittel, oberflächenaktive Verbindungen, Öle und Fette, Wachse, Parfumöle, Farbstoffe, Konservierungsmittel und spezielle Wirkstoffe.

Die gebräuchlichste Anwendungsform der erfindungsgemäßen Zubereitungen zur Behandlung von stark fettendem Haar ist das Shampoo. Derartige Shampoos können in der einfachsten Form aus der Lösung einer anionischen, kationischen, nichtionischen oder amphoteren oberflächenaktiven Verbindung in Kombination mit einer Verbindung der vorgenannten allgemeinen Formel bestehen. Als anionische Tenside können zum Beispiel in den Shampoos Alkylsulfate, Alkylethersulfate, Alkylsulfonate, sulfatierte Monoglyceride, sulfonierte Alkanolamide, Monosulfosuccinate von Fettalkoholen, Kondensationsprodukte von Fettsäuren mit Proteinhydrolysaten enthalten sein. Als kationische Tenside kommen zum Beispiel langkettige quartäre Ammoniumverbindungen, Ester aus Fettsäuren und Aminoalkoholen in Frage. Bei den in den erfindungsgemäßen Zubereitungen als nichtionische Tenside eingesetzten Produkten kann es sich zum Beispiel um Zuckerester, Ester von Polyolen, Kondensationsprodukte von Ethylenoxid mit Fettsäuren, Fettalkoholen, langkettigen Alkylphenolen, langkettigen Amiden handeln. Beispiele für einsetzbare amphotere Tenside stellen Betainderivate und Aminoxide dar. Darüber hinaus können die erfindungsgemäßen Shampoos übliche kosmetische Zusatzstoffe wie Verdickungsmittel, Farbstoffe, Duftstoffe, Konditionierungsmittel und spezielle Wirkstoffe wie Antischuppenmittel enthalten. Als Verdickungsmittel kommen dabei Fettsäurealkanolamide, Carboxymethylcellulose, Hydroxymethylcellulose, Gummen, Ester von langkettigen Polyolen in Frage.

Die erfindungsgemäßen kosmetischen Zubereitungen enthalten die Arylalkansäurederivate in einer Menge von 0,01 bis 20 Gewichtsprozent, vorzugsweise 1 bis 10 Gewichtsprozent, bezogen auf die gesamte Zubereitung. Die übrigen Bestandteile bewegen sich dabei in den für derartige Zubereitungen üblichen Größenordnungen, wie im Falle der Shampoos, zum Beispiel : Tenside 4-20 Gewichtsprozent, Verdickungsmittel 0,1 bis 5 Gewichtsprozent, Riechstoffe 0,5-2 Gewichtsprozent, Farbstoffe 0,01-0,1 Gewichtsprozent, Konservierungsmittel 0,1-0,2 Gewichtsprozent.

Mit den in Form eines Shampoos konfektionierten erfindungsgemäßen Zubereitungen werden bereits bei einmal wöchentlicher Anwendung zufriedenstellende Ergebnisse erzielt. Es gelingt hierbei, das fettige Aussehen des Haares zu vermindern und das Nachfetten in den Zeiten zwischen den Behandlungen in Grenzen zu halten,wodurch die Moglichkeit einer normalen Haarpflege gegeben ist.

Bei dem Einsatz in Form von Cremes, Milchpräparaten, Gelen gelingt es, durch regelmäßiges Auftragen auf die Haut deren Aussehen dauerhaft wesentlich zu verbessern. So ist es möglich, gegebenenfalls in Kombination mit weiteren Aknepräparaten, auch in Fällen von Akne, zu zufriedenstellenden Ergebnissen zu gelangen.

Die nachfolgenden Beispiele sollen den Gegenstand der Erfindung näher erläutern ohne ihn jedoch hierauf zu beschränken.

## Beispiele

Zunächst werden einige der in den erfindungsgemäßen kosmetischen Zubereitungen einzusetzenden Arylalkansäurederivate beschrieben.

A) 3,4-Dihydroxy-phenyl-essigsäure als Ausgangsprodukt für entsprechende Ester oder Amide

Die Herstellung der Verbindung kann gemäß den Angaben in den Berichten der deutschen chemischen Gesellschaft 42, Seite 2 949, erfolgen. Sie erfolgte im vorliegenden Fall über die als Handelsprodukt erhältliche 3,4-Dimethoxyphenylessigsäure. Die Mischung aus 115 g (587 mMol) 3,4-Dimethoxyphenylessigsäure, 1 052 g (2,35 Mol) 57 %iger Jodwasserstoffsäure und 14 g rotem Phosphor wurde 1,5 Stunden lang bei 95 °C und weitere 1,5 Stunden lang bei 105-110 °C gerührt, wobei die berechnete Menge (166,5 g) Methyljodid abdestillierte. Nach dem Abkühlen der Mischung, Abfiltrieren des roten Phosphors, Eindampfen der Lösung und Aufnahme des Rückstandes in Ether, wurde die etherische Lösung mit konzentrierter $NaHSO_3$-Lösung und Aktivkohle behandelt. Nach dem Trocknen der hellgelben Lösung mit $Na_2SO_4$ und Eindampfen wurde der Rückstand in Methylenchlorid suspendiert, restliches Wasser azeotrop abdestilliert und nach dem Abkühlen der Suspension das Festprodukt abgesaugt und bei 50 °C getrocknet. Es wurde 63 g, das sind 64 % der Theorie, 3,4-Dihydroxyphenylessigsäure vom Schmelzpunkt 126-129 °C erhalten.

B) 3,4-Dihydroxy-phenyl-essigsäureethylester

Die Verbindung wurde durch Veresterung der freien Säure mit Ethanol erhalten. $n_D^{20}$ = 1,526 8 (nicht destilliert).

C) 3,4-Dimethoxy-phenyl-essigsäure als Ausgangsprodukt für entsprechende Ester oder Amide

Die Verbindung stellt ein Handelsprodukt dar. Ihre Herstellung wird in Beilstein 10 II, Seite 268 beschrieben.

D) 3,4-Dimethoxy-phenyl-essigsäureethylester

Die Verbindung wurde durch Veresterung der freien Säure mit Ethanol erhalten. Ihre Kenndaten waren : Sdp. 112 °C/o, 13 mbar ; $n_D^{20}$ = 1,517 4. Literaturangaben finden sich im J. Chem. Soc. 1 959, Seite 2 157.

E) 2-(3,4-Dimethoxy-benzyl)-acetessigsäure-methylester

Die Verbindung ist in bekannter Weise durch Alkylierung von Acetessigsäuremethylester mit 3,4-Dimethoxy-benzylchlorid gemäß Hinweisen in Organikum, 8. Auflage, Seite 469 erhältlich. Schmp. 51 °C.

F) 4-Hydroxy-phenylessigsäure-ethylester

Die Verbindung wurde durch Veresterung der freien Säure mit Ethanol erhalten. Sdp. 117 °C/o, 14 mbar ; $n_D^{20}$ = 1,524 0. Literaturangaben : J. Org. Chem. 22 (1957) Seite 1 577

G) 3-Hydroxy-phenylessigsäure-ethylester

Die Verbindung wurde durch Veresterung der freien Säure mit Ethanol erhalten. Sdp. 111 °C/o, 16 mbar ; $n_D^{20}$ = 1,523 6. Literaturangaben : Diss. Pharm. Pharmacol 20 (1968) Seite 607.

H) 3,4-Dimethoxy-phenylessigsäure-diethylamid

Die Verbindung wurde durch Umsetzung des Säurechlorids mit Diethylamin in Ether bei Raumtemperatur erhalten. Sdp. 144 °C/0,08 mbar : $n_D^{20}$ = 1,534 2. Literaturangaben : Franz. Pat. 1 336 388.

I) 3,4-Dihydroxy-phenylessigsäure-diethylamid

Die Verbindung wurde analog dem unter A) beschriebenen Verfahren aus der Verbindung H) in 67 %iger Ausbeute hergestellt. Schmp. 134-137 °C. Literaturangaben : Helv. Chim. Acta 45 (1962) Seite 270

J) 3,4-Methylendioxy-phenylessigsäure-ethylester

Die Verbindung wurde durch Veresterung der freien Säure mit Ethanol erhalten. Sdp. 103 °C/0,13 mbar : $n_D^{20}$ = 1,519 8. Literaturangaben : Franz. Pat. 1 549 379

K) 4-(3,4-Methylendioxy-benzyloxy)-phenylessigsäure-ethylester

Zu einer Natriumalkoholatlösung von 2,16 (94 m Mol) Natrium in 100 ml Ethanol wurden unter Rühren 16,9 g 94 m Mol) 4-Hydroxy-phenylessigsäureethylester gegeben. Anschließend wurden 16,0 g (94 m Mol) 3,4-Methylendioxy-benzylchlorid zugetropft und die Mischung wurde 8 Stunden lang auf Siedetemperatur gehalten. Nach dem Eindampfen, Aufnehmen des Rückstandes in Ether, Waschen mit $Na_2CO_3$-Lösung und Wasser, Behandeln der etherischen Lösung mit Aktivkohle und Eindampfen wurde der Rückstand bei 0,13 mbar andestilliert. Bis zu einer Sumpftemperatur von 240 °C wurden 5,9 g leicht flüchtiges Material abdestilliert. Der Rückstand von 21 g wurde an Kieselgel (Merck, Korngröße 0,063-0,200 mm) mit Methylenchlorid als Eluierungsmittel säulenchromatographiert. Es wurden 16,7 g (57 % d.Th.) an 4-(3,4-Methylendioxybenzyloxy)-phenylessigsäureethylester vom Schmelzpunkt 49-52 °C und Brechungsindex $n_D^{20}$ = 1,564 3 erhalten.

L) 3-(3,4-Dimethoxy-phenyl)-propionsäureethylester

Die Verbindung wurde durch Veresterung der freien Säure mit Ethanol erhalten. Sdp. 119 °C/0,16 mbar, $n_D^{20}$ : 1,513 7. (Literaturangaben finden sich im J. Chem. Soc. 1929, Seite 2 014).

M) N-[β-(3,4-Dimethoxy-phenyl)-ethyl]-3,4-dimethoxyphenylacetamid

Die Verbindung wurde nach Angaben in Ber. dtsch. chem. Ges. 42 (1909) Seite 1986 durch Reaktion des Säurechlorids mit β-(3,4-Dimethoxy-phenyl)-ethylamin in Gegenwart von Triethylamin erhalten : Schmp. 122-124 °C.

N) N-[β-(3,4-Dimethoxy-phenyl)-ethyl]-N-methyl-3,4-dimethoxy-phenylacetamid

Die Verbindung wurde durch Methylierung von M) mit Methyljodid als gelbes Öl vom Brechungsindex $n_D^{20}$ : 1,568 0 erhalten.

O) 3,4-Dimethoxy-phenylessigsäure-3,4-dimethoxy-benzylester

Die Verbindung wurde durch Reaktion des Säurechlorids mit 3,4-Dimethoxy-benzylalkohol erhalten. Schmp. 99-101 °C.

Die antiseborrhoeische Wirkung der in den erfindungsgemäßen kosmetischenZubereitungen eingesetzten Verbindungen wurde durch nachfolgend beschriebene Tierversuche näher untersucht. Als Versuchstiere dienten männliche Wistar-Ratten von 220-230 g Körpergewicht. Beurteilt wurde visuell der Bräunungsgrad auf dem Rücken der dort geschorenen Ratten. Die Bräunung wird durch das braune Hautoberflächenlipid der Ratten hervorgerufen. Dieser Test geht von der Beobachtung aus, daß junge weibliche Ratten sowie männliche Ratten nach dem Waschen mit Tensidlösung beziehungsweise einem Lipidlösungsmittel und auch männliche Ratten, die systemisch mit Östrogenen behandelt wurden, nur die normale helle, rosafarbene Haut nach dem Scheren aufweisen ; parallel dazu sind aus den abgeschnittenen Haaren nur noch vergleichsweise sehr geringe Lipidmengen zu extrahieren.

Zur Beurteilung der sebosuppressiven Wirksamkeit wurden die Prüfsubstanzen A, B, D, E, G und H in Form einer 1 %igen Lösung in Ehtanol oder Ethanol/Aceton(1 : 1) jeweils 6 Ratten halbseitig auf das Rückenfell gepinselt. Die andere Seite wurde nur mit dem Lösungsmittel ohne Wirksubstanz behandelt (Kontrollseite). Während der Versuchsdauer von 14 Tagen wurde an insgesamt 9 Tagen einmal appliziert. Zur weiterenKontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unabhängig unter Doppelblindbedingungen visuell abgemustert.

Als 1. Kriterium wurde bewertet, ob die Mehrheit der Beurteiler richtig die behandelte Seite erkannt haben, wobei wie folgt differenziert wurde :

| Zeichen | Anteil Beurteiler, die eine Wirkung erkannten |
|---|---|
| ++ | 100 % |
| + | > 50 %, 100 % |
| 0 | ≤ 50 % |

Als zweites Kriterium wurde der Unterschied zwischen rechter und linker Seite gewertet, wobei pro Beurteiler und Tier jeweils 1 Punkt zu vergeben war, und zwar in der Weise, daß die dunklere Seite mit 1 und die hellere mit 0 und bei Gleichheit beide Seiten mit 0,5 benotet wurden.

Als drittes Kriterium wurden außerdem noch die Intensitätsunterschiede der Brauntöne nach folgender Skala bewertet :

3 Punkte stark braun
2 Punkte mittel braun
1 Punkt schwach braun
0 Punkte keine Braunfärbung

Signifikante Differenzen zwischen unbehandelter und behandelter Seite nach der zweiten Bewertungsmethode zeigen die lokale Wirksamkeit einer Substanz an. Nach der dritten Bewertungsmethode werden die Punktsummendifferenzen zwischen den unbehandelten Kontrolltieren und jeweils den behandelten und unbehandelten Seiten der Versuchstiergruppe gebildet, wobei wiederum signifikante Differenzen zwischen Kontrolltieren und der behandelten Seite der Versuchstiere die Wirkung einer Substanz deutlich machen. Parallel dazu ist in der Regel auch ein deutlicher Unterschied zwischen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische. Substanzübertragung von einer auf die andere Seite oder Lösungsmitteleinfluß. Zur Differenzierung der Effekte gemäß Beurteilungsmethode 2 und 3 wurde das folgende Schema verwendet.

| Zeichen | Punktdifferenz |
|---|---|
| ++ | sehr groß (> 99,9 % Wahrscheinlichkeit) |
| + | signifikant (≥ 95 % Wahrscheinlichkeit) |
| (+) | deutlich, aber < 95 % Wahrscheinlichkeit |

Die nachfolgende Tabelle 1 zeigt die Bewertungsergebnisse nach vorgenanntem Schema für die untersuchten Substanzen.

Tabelle 1

Bewertung der sebosuppressiven Effekte

| Substanz | Bewertungsmethode 1 | 2 | 3 |
|---|---|---|---|
| B | 0 | (+) | + |
| D | + | ++ | ++ |
| E | + | (+) | + |
| G | 0 | + | + |
| H | 0 | (+) | + |
| L | + | + | + |
| M | ++ | + | + |
| N | 0 | + | (+) |
| O | + | (+) | + |

Nachfolgend werden Beispiele für erfindungsgemäße topische kosmetische Zubereitungen zur Behandlung von stark fettendem Haar seborrhoeischer Haut angegeben.

Hautemulsion zur Behandlung fetter Haut

| | |
|---|---|
| Kolloiddisperses Gemisch von Cetylstearylalkohol, Natrium-Cetylstearylsulfat und nichtionogenem Emulgator, Emulgade F(R), Dehydag | 1,00 Gewichtsteile |
| Gemisch aus Cetylstearylalkohol und nichtionogenem Emulgator, Emulgade F spez. (R) Dehydag | 1,00 Gewichtsteile |
| Ölsäuredecylester | 1,00 Gewichtsteile |
| Stearinsäure | 3,00 Gewichtsteile |
| Wollfett, wasserfrei | 0,50 Gewichtsteile |

| | |
|---|---|
| Glycerin | 1,00 Gewichtsteile |
| Triethanolamin | 0,25 Gewichtsteile |
| Verbindung B | 3,25 Gewichtsteile |
| Wasser | 89,00 Gewichtsteile |

Zur Verwendung der Hautemulsion in Aerosolform werden 92 Gewichtsteile der Emulsion mit 8 Gewichtsteilen Treibgas in einem Aerosolbehälter abgefüllt.

Creme zur Behandlung fetter Haut

| | |
|---|---|
| Selbstemulgierendes Gemisch aus Mono/Diglyceriden höherer gesättigter Fettsäuren mit Kaliumstearat(R) Dehydag | 16,0 Gewichtsteile |
| Cetylstearylalkohol mit ca. 12 Mol Ethylenoxid | 1,0 Gewichtsteile |
| 2-Octyldodecanol | 6,0 Gewichtsteile |
| Isopropylmyrisat | 4,0 Gewichtsteile |
| Glycerin | 6,0 Gewichtsteile |
| Verbindung D | 7,0 Gewichtsteile |
| Wasser | 60,0 Gewichtsteile |

Shampoo für fettendes Haar

| | |
|---|---|
| Triethanolaminlaurylsulfat mit 42 % Waschaktivsubstanz | 12,0 Gewichtsteile |
| Kokosfettsäurediethanolamid | 2,0 Gewichtsteile |
| Carboxymethylcellulose | 0,25 Gewichtsteile |
| Verbindung E | 8,25 Gewichtsteile |
| Parfumöl | 0,2 Gewichtsteile |
| Wasser | 77,3 Gewichtsteile |

**Patentansprüche**

1. Topische kosmetische Zubereitungen mit einem Gehalt an Verbindungen der allgemeinen Formel

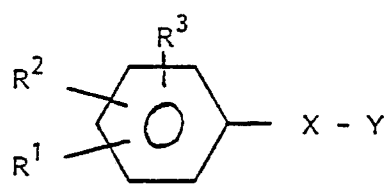

in der $R^1$, $R^2$ und $R^3$ unabhängig voneinander für ein Wasserstoffatom, eine Hydroxylgruppe, eine $OR^4$-Gruppe, wobei $R^4$ einen Alkylrest mit 1-6 Kohlenstoffatomen bedeutet, oder zwei der Reste $R^1$, $R^2$ und $R^3$ für eine Methylendioxygruppe stehen, wobei die Reste $R^1$, $R^2$ und $R^3$ nicht gleichzeitig Wasserstoff bedeuten, X für eine Alkylengruppe mit 1-3 Kohlenstoffatomen in der Kette und Y für eine Gruppe $CONR^5R^6$ oder $COOR^7$ stehen, worin $R^5$ und $R^6$ ein Wasserstoffatom, einen Alkylrest mit 1-6 Kohlenstoffatomen, einen Benzyl- oder 3,4-Dimethoxybenzylrest und $R^7$ einen Alkylrest mit 1-6 Kohlenstoffatomen darstellen, oder an
2-(3,4-Dimethoxy-benzyl)-acetessigsäure-methylester
4-(3,4-Methylendioxy-benzyloxy)-phenylessigsäureethylester
N-[β-(3,4-Dimethoxy-phenyl)-ethyl]-3,4-dimethoxy-phenyl-acetamid oder
N-[β-(3,4-Dimethoxy-phenyl)-ethyl]-N-methyl-3,4-dimethoxy-phenylacetamid

2. Topische kosmetische Zubereitungen nach Anspruch 1, dadurch gekennzeichnet, daß sie die Verbindungen vorgenannter Struktur in einer Menge von 0,01 bis 20 Gewichtsprozent, vorzugsweise 1-10 Gewichtsprozent, bezogen auf die gesamte Zubereitung, enthalten.

**Claims**

1. Topical cosmetic preparations containing compounds corresponding to the following general formula

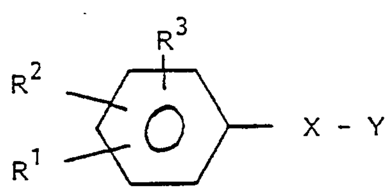

in which $R^1$, $R^2$ and $R^3$ independently of one another represent a hydrogen atom, a hydroxyl group, an $OR^4$-group, where $R^4$ is a $C_1$-$C_6$ alkyl group, or two of the radicals $R^1$, $R^2$ and $R^3$ represent a methylenedioxy group, the radicals $R^1$, $R^2$ and $R^3$ not all representing hydrogen at the same time, X represents an alkylene group containing from 1 to 3 carbon atoms in the chain and Y represents a group $CONR^5R^6$ or $COOR^7$, where $R^5$ and $R^6$ represent a hydrogen atom, an alkyl group containing from 1 to 6 carbon atoms, a benzyl or 3,4-dimethoxybenzyl radical and $R^7$ represents an alkyl group containing from 1 to 6 carbon atoms, or

2-(3,4-dimethoxybenzyl)-acetoacetic acid methylester,
4-(3,4-methylenedioxy-benzyloxy)-phenylacetic acid ethylester,
N-[β-(3,4-dimethoxyphenyl)-ethyl]-3,4-dimethoxyphenylacetamide or
N-[β-(3,4-dimethoxyphenyl)-ethyl]-N-methyl-3,4-dimethoxyphenylacetamide.

2. Topical cosmetic preparations as claimed in Claim 1, characterized in that they contain the compounds having the above-illustrated structure in a quantity of from 0.01 to 20 % by weight and preferably in a quantity of from 1 to 10 % by weight, based on the preparation as a whole.

**Revendications**

1. Préparations cosmétiques topiques ayant une teneur en composés de formule générale :

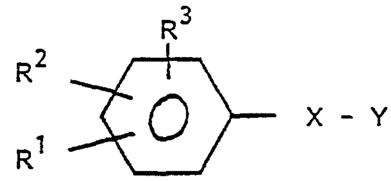

dans laquelle $R^1$, $R^2$ et $R^3$ indépendamment les uns des autres représentent un atome d'hydrogène, un groupe hydroxyle, un groupe $OR^4$ avec $R^4$ signifiant un radical alcoyle ayant 1 à 6 atomes de carbone, ou dans laquelle deux des radicaux $R^1$, $R^2$ et $R^3$ représentent un groupe méthylènedioxy, les radicaux $R^1$, $R^2$ et $R^3$ ne pouvant pas signifier simultanément de l'hydrogène, X représente un groupe alcoylène ayant 1 à 3 atomes de carbone dans la chaîne et Y un groupe $CONR^5R^6$ ou $COOR^7$, $R^5$ et $R^6$ représentant un atome d'hydrogène, un radical alcoyle ayant 1 à 6 atomes de carbone, un radical benzyle ou 3,4-diméthoxybenzyle et $R^7$ un radical alcoyle ayant 1 à 6 atomes de carbone, ou bien ayant une teneur en

2-(3,4-diméthoxy-benzyl)-acétoacétate de méthyle
4-(3,4-méthylènedioxy-benzyloxy)-phénylacétate d'éthyle
N-[β-(3,4-diméthoxy-phényl)-éthyl]-3,4-diméthoxyphénylacétamide ou
N-[β-(3,4-diméthoxy-phényl)-éthyl]-N-méthyl-3,4-diméthoxy-phénylacétamide.

2. Préparations cosmétiques topiques selon la revendication 1, caractérisées en ce qu'elles contiennent les composés de structure précitée en une quantité de 0,01 à 20 % en poids, de préférence de 1 à 10 % en poids par rapport à la préparation totale.